Europäisches Patentamt

European Patent Office

Office européen des brevets

(11) Publication number: **0 257 368 B1**

# EUROPEAN PATENT SPECIFICATION

(45) Date of publication of patent specification: **25.03.92**

(51) Int. Cl.⁵: **A61K 37/00**, A61K 37/02, A61K 37/36, A61K 9/14, A61K 9/10

(21) Application number: **87111217.3**

(22) Date of filing: **04.08.87**

(54) Compositions for parenteral administration and their use.

(30) Priority: **11.08.86 US 895608**

(43) Date of publication of application:
**02.03.88 Bulletin 88/09**

(45) Publication of the grant of the patent:
**25.03.92 Bulletin 92/13**

(84) Designated Contracting States:
**AT BE CH DE ES FR GB GR IT LI LU NL SE**

(56) References cited:
**EP-A- 0 085 036**
**EP-A- 0 205 051**
**DE-B- 2 406 621**

(73) Proprietor: **AMERICAN CYANAMID COMPANY**
**1937 West Main Street P.O. Box 60**
**Stamford Connecticut 06904-0060(US)**

(72) Inventor: **Steber, William**
**30 Mooney Road**
**Ledgewood, NJ 07852(US)**
Inventor: **Fishbein, Richard**
**18 Meadow Run Drive**
**Skillman, NJ 08558(US)**
Inventor: **Cady, Susan Mancini**
**1501 Revere Road**
**Yardley, PA 19067(US)**

(74) Representative: **Wächtershäuser, Günter, Dr.**
**Tal 29**
**W-8000 München 2(DE)**

**Description**

The invention is a microsphere composition of a fat or wax or mixtures thereof and a biologically active protein, peptide or polypeptide which is administered by dispersion in a pharmaceutically and pharmacologically acceptable liquid vehicle. The invention is also a slow release composition of a fat or wax or mixtures thereof and a biologically active protein, peptide or polypeptide dispersed in a pharmaceutically and pharmacologically acceptable liquid vehicle. There may be hydrophobic interaction, bonding or coating of the active ingredient to the fat or wax.

The compositions are suitable for parenteral administration to animals. The term "biologically active proteins, peptides or polypeptides" comprise growth hormones, somatomedins, growth factors, and other biologically active fragments or derivatives thereof.

The invention includes a method for increasing milk production in animals, particularly dairy cows comprising parenterally administering compositions of the invention to the cows. The invention also includes a method for elevating and maintaining elevated blood levels of biologically active proteins, peptides or polypeptides in humans or animals comprising parenterally administering compositions of the inventions.

EP-A-0 085 036 describes increased production of milk from cows by administering to cows an effective amount of biosynthetic growth hormone or analogs thereof. The citation fails to contain any description of a specifically suitable system to be used for slow release, let alone a description of a fat or wax delivery system as described and claimed by the present invention.

Waxes and fats which are suitable for use in the compositions of this invention in general have melting points higher than 40°C. The wax of the invention may be defined as set forth in Hawley's The Condensed Chemical Dictionary, Eleventh Edition, as a low-melting organic mixture or compound of high molecular weight, solid at room temperture and generally similar in composition to fats and oils except that it contains no glycerides. Some are hydrocarbons; others are esters of fatty acids and alcohols. These compounds include saturated or unsaturated long chain $C_{10}$-$C_{24}$ fatty acids, alcohols, esters, salts, ethers or mixtures thereof. They are classed among the lipids. Waxes are thermoplastic, but since they are not high polymers, they are not considered in the family of plastics. Common properties are water repellency; smooth texture; nontoxicity; freedom from objectionable odor and color. They are combustible and have good dielectric properties. They are soluble in most organic solvents and are insoluble in water. The major types are as follows:

I. Natural

    1. Animal (beeswax, lanolin, shellac wax, Chinese insect wax)
    2. Vegetable (carnauba, candelilla, bayberry, sugar cane)
    3. Mineral
        (a) Fossil or earth waxes (ozocerite, ceresin, montan)
        (b) petroleum waxes (paraffin, microcrystalline)(slack or scale wax)

II. Synthetic

    1. Ethylenic polymers and polyol ether-esters ("Carbowax," sorbitol)
    2. Chlorinated naphthalenes ("Halowax")
    3. Hydrocarbon type via Ficher-Tropsch synthesis

The fat of the invention may be defined as set forth in Hawley's The Condensed Chemical Dictionary, Eleventh Edition, as a glyceryl ester of higher fatty acids such as stearic and palmitic. Such esters and their mixtures are solids at room temperatures and exhibit crystalline structure. Lard and tallow are examples. There is no chemical difference between a fat and an oil, the only distinction being that fats are solid at room temperature and oils are liquid. The term "fat" usually refers to triglycerides specifically, whereas "lipid" is all-inclusive.

The fat is preferably composed of mono-, di-or triglyceryl esters of long chain $C_{10}$-$C_{24}$ fatty acids. The mono-, di-, or triglycerides are composed predominantly of stearates, palmitates, laurates, linoleates, linolenates, oleates, and residues or mixtures thereof, having melting points greater than 50°C being most preferred. Glyceryl tristearate is a most preferred fat. Additionally, lipophilic salts of fatty acids such as magnesium stearate and the like are also suitable.

The microspheres of the invention are dispersed in a pharmaceutically and pharmacologically acceptable liquid to obtain a slow release composition for parenteral administration. The vehicle may be aqueous buffered systems or oil systems. The oil may be derived from animal or vegetable sources or be synthetic.

Preferred oils include neutral mono-, di-or triglyceride liquid or mixtures thereof. A neutral oil is one containing no residual acid. Vehicles suitable for use in the compositions of this invention include aqueous systems such as buffered salines; organic solvents such as glycols and alcohols; and water immiscible liquids such as oils, depending upon the solubility of the active ingredient being administered.

The biologically active proteins, peptides and polypeptides suitable for administration in the compositions of the invention comprise growth hormones, somatomedins, growth factors, and other biologically active fragments and derivatives thereof. Preferred proteins include bovine, ovine, equine, porcine, avian, and human growth hormones; and is meant to encompass those which are of natural, synthetic, recombinant or biosynthetic origin. Additionally, metals or metal compounds associated with biologically active proteins, peptides and polypeptides, as well as acid salts, derivatives and complexes and antihydrating agents are suitable for incorporation into the compositions of the invention.

Stabilizers, preservatives, surfactants, salts, buffers or mixtures thereof may be included in the compositions of the invention. Preferred stabilizers include dehydroacetic acid, salicylanilide, sorbic acid, boric acid, benzoic acid, and salts thereof; sodium nitrite and sodium nitrate. The amounts of said materials suitable for use in the invention range from about 0.1% to 20% on a weight basis.

Preferred surfactants for use in compositions of the invention containing biologically active macromolecules are non-ionic in nature such as polyoxyethylene sorbitan mono-oleate (20 moles ethoxylation), and block copolymers of ethylene oxide and propylene oxide. The amounts of surfactants suitable for use in the invention range from about 0.1% to 10.0% on a weight basis being preferred.

Uniquely, it has been found that increased blood levels of growth hormones may be obtained and maintained for extended periods of time, as can increased weight gains and increased milk production in lactating animals by injecting animals with the compositions of the invention in a suitable vehicle. Elevated blood levels of the biologically active proteins, peptides and polypeptides are generally observed and associated with beneficial and/or therapeutic effects. The effects include weight gain, increased growth rate, increased milk production in lactating animals and associated increased availability milk to nursing offspring of treated animals, improved muscle size, improved feed efficiency, decreased body fat and improved the lean meat to fat ratio. Maintaining the elevated blood levels is an indication of the slow release of the active ingredient. Properties such as increased milk production, growth rate, improved feed efficiency and increased lean meat are generally observed when elevated blood levels of the active ingredient are maintained. The invention includes the use of the compositions herein to improve milk production, increase growth rate, improve feed efficiency, increase lean meat in animals, increase and maintain levels of hormones in the blood stream of animals.

A preferred embodiment of this invention involves the incorporation of the biologically active protein, peptide or polypeptide in fat or wax microspheres which may optionally also contain some or all of the excipients described above, which are then dispersed in the vehicle. The microspheres, preferably fat microspheres, may be up to 1,000 microns in diameter, with a weight average size range of 25 microns to 300 microns being preferred for parenteral administration. Microspheres containing up to about 70% of a biologically active protein, peptide or polypeptide exhibit sustained release for various periods of time depending upon the solubility of the active ingredient and the nature of the wax or fat, surfactant, buffer and vehicle employed.

The invention in its broadest sense is a slow release composition comprising a mixture of the fat or wax or mixture thereof and a biologically active protein, peptide or polypeptide drug dispersed in a pharmaceutically and pharmacologically acceptable vehicle. Microspheres and coated protein particles may be present in the slow release composition. The composition may be the protein dissolved in the fat or wax or there may be hydrophobic interaction or bonding of the active ingredient to the fat or wax. For the administration of hormone, compositions comprising on a weight basis about 1% to 30% of growth hormone, somatomedin, growth factor or biologically active fragment or derivative thereof preferably having a weight average particle size less than 20 microns; about 5% to 60% and preferably about 10% to 48% of the fat, wax or mixture thereof; optionally containing up to about 15% of excipients such as surfactants, stabilizers, preservatives, salts or buffers or mixtures thereof with sufficient amount of a pharmaceutically and pharmacologically acceptable liquid vehicle to total 100%. The vehicle is again the aqueous buffered or oil system described above.

For the parenteral administration of growth hormones such as bovine growth hormone, microspheres comprising on a weight basis of about 5% to 40% of the solid hormone preferably having a weight average particle size of less than 20 microns, containing up to about 20% of the other excipients as described above in about 40% to 95% of fat or wax or a mixture thereof; having a weight average particle size range of 25 microns to 300 microns have demonstrated sustained release of the hormone and sustained increases in milk production in lactating dairy cows for about two weeks and are preferred. These preferred compositions have demonstrated increased milk production which is comparable to that obtained by daily injection of bovine growth hormone.

For the administration of compositions containing water soluble proteins, peptides or polypeptides such as bovine growth hormone, water immiscible liquids are preferred, with oils or liquid fats and water immiscible alcohols and glycols and mixtures thereof being more preferred. The vehicles are chosen so as to both disperse and coat the mixture or microspheres and also to provide an acceptable viscosity of the injection mixture using HLV values (Hydrophilic Lipophilic Balance) and viscosity as criteria for their selection.

On this basis, fatty acid glycerides and blends thereof which are liquid at ambient temperatures including synthetic oils; vegetable oils, such as olive, sesame seed, peanut, sunflower seed, soybean, cottonseed, corn, safflower, palm, rapeseed and coconut; animal oils such as fish oils, fish liver oils, sperm oils; or fractions derived therefrom; and mixtures thereof; having HLB values in a range of 1 to 5 and viscosities in a range of from 10 cps to 1000 cps as measured with a Brookfield viscometer RTV using a #1 spindle; find utility as vehicles for compositions of the present invention.

The microspheres of the invention may be prepared by incorporating the active ingredient, having the desired particle size, and other excipients with a molten fat, wax or mixture thereof and then forming microspheres of the resulting mixture by a variety of techniques such as emulsifying or atomizing the mixture or by processing the mixture of ingredients and molten fat, wax or mixture thereof mechanically and cooling, for example utilizing a centrifugal disc, as described in International Patent Application PCT/US 85/00827. Alternatively, the mixture of active ingredients, excipients and fat, waxes and mixtures thereof may be cooled to give a solid which may then be processed by procedures such as milling, grinding and the like.

Mixtures of the invention suitable for injection are readily prepared by dispersing the protein, peptide or polypeptide, excipients, fat, wax or mixture thereof at elevated temperatures directly in the vehicle and cooling. The invention is further illustrated by the following non-limiting examples.

Example 1

Preparation of injectable microspheres for the parenteral administration of bovine growth hormone.

1. Preparation of bovine growth hormone and additives in a size range suitable for incorporation in microspheres by spray drying may be accomplished by dissolving bovine growth hormone in dilute ammonium hydroxide solution and then adding the desired salt solutions such as sodium benzoate. A nonionic surfactant such as a block copolymer of ethylene oxide and propylene oxide is added and allowed to dissolve with constant gentle mixing. The solution is then spray-dried in a Buchi mini spray dryer, model #190.

2. Preparation of injectable microspheres. A homogeneous mixture of active ingredient and additives in the molten fat, wax or mixture thereof is prepared and the resulting mixture sprayed through an air/liquid spray nozzle equipped with a heated jacket to maintain the incoming air and the molten phase at a temperature above the melting point. The microspheres are formed as the molten droplets cool and are collected on a series of sieves in the desired size range of about 45 to 180 microns and retained for use. Those microspheres which are not of the desired size range are collected for recycling. Alternatively, the homogeneous mixture may be fed onto a centrifugal disc and the microspheres thus formed are collected as above, or the molten mixture may be cooled and milled to the desired average particle size range.

Utilizing the above procedure with the materials listed in Table I below yields the injectable microsphere compositions listed in Table II below:

Table I

| Name | Designation |
|---|---|
| Glyceryl Monostearate | GMS |
| Glyceryl Distearate | GDS |
| Glyceryl Tristearate | GTS |
| Glyceryl Trilaurate | GTL |
| Soya Oil | Soya Oil |
| Beeswax | Beeswax |
| Carnauba Wax | Carnuaba |
| Sodium Laurate | NaLaurate |
| Sodium Benzoate | NaBenzoate or NaBz |
| Sodium Carbonate | NaCarb |
| Sodium Bicarbonate | BiCarb |
| Calcium Benzoate | CaBenzoate |
| Polyoxyethylene Sorbitan Monooleate (20 moles of ethoxylation, Tween® 80[1]) | Surf. A |
| Block copolymer of ethylene oxide and propylene oxide, (Pluronic® F68[2]) | Surf. B |
| Glyceryl dioleate | GDO |
| Polyoxyethylene Stearate, 23 moles of ethoxylation | POE (23) stearate |

[1]Trademark of ICI Americas, Inc.

[2]Trademark of BASF Wyandotte Corp.

5

Table I (Continued)

| Name | Designation |
|---|---|
| Polyethylene Glycol Distearate, average MW of PEG 1540 | PEG 1540 distearate |
| Polyethylene Glycol, average MW 6000 | PEG 6000 |
| Polyoxyethylene Sorbitan Monostearate | POE (20) sorbitan monostearate |
| Carbonate Buffered Saline (pH 9.4) | CBS |
| Polyethylene glycol | PEG |
| Mixture of caprylic, capric, lauric and caproic triglycerides | Miglyol® 812[3] |

[3]Trademark of Dynamit Nobel

6

EP 0 257 368 B1

Table II

Microsphere Compositions

| Composition # | Matrix | BGH (%) | Additives(%) Buffer/Pres. | Surfactant |
|---|---|---|---|---|
| 1 | GMS | 2.5 | -- | -- |
| 2 | | 7.5 | -- | -- |
| 3 | | 20 | -- | -- |
| 4 | GMS/soya oil | 20 | -- | -- |
| 5 | | 30 | -- | -- |
| 6 | GDS | 10 | -- | -- |
| 7 | | 10 | NaLaurate(1.4) | -- |
| 8 | | 25 | NaBenzoate(1.6) | Surf.B(0.12) |
| 9 | | 25 | NaCarb/bicarb(1.3) | Surf.A(0.18) |
| 10 | GTS/GDS (19:1) | 10 | -- | -- |
| 11 | | 15 | -- | -- |
| 12 | | 10 | NaBenzoate(0.6) | Surf.B(0.05) |
| 13 | GTS/GDS (9:1) | 10 | -- | Surf.B(0.1) |
| 14 | | 10 | NaBenzoate(0.6) | Surf.B(0.05) |

Table II (Continued)

Microsphere Compositions

| Composition # | Matrix | BGH (%) | Additives(%) | |
|---|---|---|---|---|
| | | | Buffer/Pres. | Surfactant |
| 15 | | 18 | -- | -- |
| 16 | | 15 | -- | -- |
| 17 | | 15 | CaBenzoate(1.1) | -- |
| 18 | GTS/beeswax (9:1) | 7.5 | -- | -- |
| 19 | | 15 | -- | -- |
| 20 | GTS/GMS (19:1) | 7.5 | -- | -- |
| 21 | | 12 | -- | -- |
| 22 | | 15 | NaBenzoate(1.4) | -- |
| 23 | GTS/soya oil (19:1) | 7.5 | -- | -- |
| 24 | (39:1) | 7.5 | -- | -- |
| 25 | GMS/carnauba (1:2) | 20 | -- | -- |
| 26 | GTS | 7.5 | -- | -- |
| 27 | | 10 | -- | -- |
| 28 | | 15 | -- | -- |

EP 0 257 368 B1

Table II (Continued)

Microsphere Compositions

| Composition # | Matrix | BGH (%) | Additives(%) | |
| | | | Buffer/Pres. | Surfactant |
|---|---|---|---|---|
| 29 | | 10 | NaBenzoate(0.8) | -- |
| 30 | | 10 | -- | Surf.B(0.05) |
| 31 | | 10 | -- | Surf.B(0.2) |
| 32 | | 25 | -- | -- |
| 33 | | 25 | NaBenzoate(0.75) | Surf.B(0.12) |
| 34 | | 25 | NaBenzoate(1.6) | Surf.B(0.12) |
| 35 | | 25 | NaBenzoate(3.2) | Surf.B(0.12) |
| 36 | | 25 | NaBenzoate(1.6) | -- |
| 37 | | 25 | -- | Surf.B(0.12) |
| 38 | | 25 | -- | Surf.B(0.24) |
| 39 | | 25 | -- | Surf.A(0.12) |
| 40 | | 25 | -- | Surf.A(0.24) |
| 41 | | 25 | -- | Surf.A(0.48) |
| 42 | | 25 | NaCarb/bicarb(1.2) | -- |

Table II (Continued)

Microsphere Compositions

| Composition # | Matrix | BGH (%) | Additives(%) | |
| --- | --- | --- | --- | --- |
| | | | Buffer/Pres. | Surfactant |
| 43 | | 25 | NaCarb/bicarb(1.2) | Surf.A(0.12) |
| 44 | | 33 | NaCarb/bicarb(2.1) | Surf.A(0.17) |
| 45 | | 33 | NaBenzoate(2.1) | Surf.B(0.17) |
| 46 | | 33 | NaBenzoate(2.1) | Surf.A(0.17) |
| 47 | GTL | 33 | NaBenzoate(2.1) | Surf.A(0.17) |
| 48 | GTS/GDS(12:1) | 15 | CaBenzoate(1.1) | -- |
| 49 | Beeswax/Soya Oil (2:1) | 30 | -- | -- |
| 50 | GMS/Soya Oil (47.25/24) | 25 | -- | Sorbitan monoleate (3.75) |
| 51 | GMS | 20 | -- | PEG 1540 distearate(16.0) |
| 52 | Diethylene glycol monostearate | 25 | -- | -- |

EP 0 257 368 B1

### Table II (Continued)
### Microsphere Compositions

| Composition # | Matrix | BGH (%) | Additives(%) Buffer/Pres. | Additives(%) Surfactant |
|---|---|---|---|---|
| 53 | GMS | 10 | -- | POE(23) stearate (9.0) |
| 54 | GMS | 20 | -- | PEG 6000(9.0) |
| 55 | GMS | 20 | -- | PEG 6000(4.0) |
| 56 | GMS | 20 | -- | PEG 6000(0.8) |
| 57 | GMS | 20 | -- | POE (20) Sorbitan monostearate (4.0) |

EP 0 257 368 B1

## Table II (Continued)
### Microsphere Compositions

| Composition # | Matrix | BGH (%) | Buffer/Pres. | Additives(%) Surfactant |
|---|---|---|---|---|
| 58 | GMS | 20 | -- | PEG 400 distearate (8.0) |
| 59 | GMS | 20 | -- | PEG 400 distearate (1.57) |
| 60 | GMS | 30 | -- | Sorbitan tristearate (7) |

Example 2

Effectiveness of injectable microspheres in various vehicles.

The efficacy of various injectable compositions of microspheres in different vehicles is determined utilizing a hypophysectomized (hypox) rat assay. The hypophysectomized rat does not produce its own growth hormone and is sensitive to injected bovine growth hormone. The response measured is growth over a period of time such as 10 days.

Each of the hypox albino rats (Taconic Farms, Sprague Dawley derived) is injected with a sufficient quantity of microsphere compositions prepared in Example 1 to provide a 10-day dose of 800 micrograms (80 micrograms/day) of bovine growth hormone in 0.2 ml of the vehicles listed in Table III below.

Table III

| Vehicle Components | |
|---|---|
| Name | Designation |
| Glyceryl Dioleate | GDO |
| Saline | SAL |
| Capric/Caprylic Triglycerides | C/C |
| Soya Oil | Soya Oil |
| Diethyl Succinate | DES |
| Aluminum Monostearate Gel | Gel |
| Carbowax (Thickener) | THX |

Test Procedures

Prior to the test, the animals are weighed and the animals to be used for the test are selected based on body weight. Only those animals whose body weights are one standard deviation from the mean body weight of the group are selected. The resulting group is then randomly divided into treatment groups consisting of eight rats/group by a computer generated randomization procedure. The test animals are then transferred to a clean cage and housed four rats/cage. On the initial day of the study the test animals are weighed and any animals with excessive weight gain or loss (±5 grams) are replaced. The animals are then assigned to test groups and treated.

At the end of the ten-day test period, total weight gain for each animal is recorded and the average weight gain per rat for each treatment determined. The results of these experiments, which are summarized in Table IV below, demonstrate the effectiveness of injectable composition of microspheres with various vehicles.

13

Table IV

| Efficacy of Injectable Microspheres in Various Vehicles | | |
|---|---|---|
| Microsphere Composition of Example[1] | Vehicle | 10-Day Growth (Grams) |
| 48 | GDO | 19.5 |
| 32 | GDO | 17.9 |
| 18 | SAL | 19.4 |
| 16 | GDO | 18.5 |
| 12 | C/C | 19.8 |
| 16 | GDO | 15.5 |
| 12 | SAL | 16.6 |
| 27 | SAL | 17.4 |
| 16 | C/C | 14.0 |
| 16 | GDO/DES (4:1) | 13.7 |
| 32 | Soya Oil | 15.6 |
| 39 | Soya Oil | 16.0 |
| 8 | 8% Gel/Soya Oil | 14.0 |
| 34 | 8% Gel/Soya Oil | 8.0 |
| 42 | Soya Oil | 12.6 |
| 43 | Soya Oil | 8.0 |
| 34 | 0.5% THX/Soya Oil | 17.0 |
| 37 | 0.5% THX/Soya Oil | 14.0 |
| 36 | 0.5% THX/Soya Oil | 13.6 |
| 33 | 0.5% THX/Soya Oil | 16.8 |
| 35 | 0.5% THX/Soya Oil | 14.5 |
| | negative controls[*] | 0.7±3.15 |

* average growth of 22 negative control groups (no treatment)

Example 3

Evaluation of growth hormone microsphere compositions in dairy cows.

Lactating cows are divided into groups of three to eight animals. Throughout the test, all cows are fed the same ration of corn silage, alfalfa hay and dairy concentrate adequate to produce 25 kg to 30 kg of milk per day. The cows are not treated for two weeks and daily milk production and bovine growth hormone blood levels obtained for each group of animals. The experimental or control treatments listed in Table V below are then administered during the treatment period. Growth hormone levels in the blood of the animals is determined by standard RIA techniques periodically and milk production levels are recorded daily. The results of these experiments, which are summarized in Tables VI and VII below, demonstrate the effectiveness of the compositions of the invention for increasing milk production, and for increasing and maintaining elevated growth hormone levels in the blood respectively.

Table V: Formulations Used in Dairy Cows

Microsphere Composition %

| # | bGH | Preservative/Stabilizer/ Surfactant | Matrix | Vehicle | MS/Vehicle Ratio | bGH Dose |
|---|-----|-------------------------------------|--------|---------|------------------|----------|
| A | -- | -- | -- | CBS | -- | 12.5 mg for 28 days |
| B | 24.5 | NaBz(1.6)/Surf. B(0.12) | GTS(73.8) | Miglyol/ Soya Oil (4/1) | 1/2.5 | 350 mg day 0, day 14 |
| C | 24.5 | NaBz(1.6)/Surf. B(0.12) | GTS(73.8) | Miglyol/ Soya Oil (4/1) | 1/2.5 | 525 mg day 0, day 14 |
| D | -- | -- | -- | CBS | | 25 mg for 28 days |
| E | 24.5 | NaCarb/Bicarb (1/2)/ Surf. A(0.12) | GDS(74.3) | Soya Oil | 1/2.5 | 350 mg day 0, day 14 |
| F | 24.5 | NaBz(1.6)/Surf. B(0.12) | GTS(73.8) | Miglyol/ Soya Oil (4/1) | 1/2.5 | 350 mg day 0, day 14 |
| G | -- | -- | -- | CBS | | 25 mg for 14 days |

EP 0 257 368 B1

Table V: Formulations Used in Dairy Cows (Continued)

| | | Microsphere Composition % | | | MS/Vehicle | |
| # | bGH | Preservative/Stabilizer/ Surfactant | Matrix | Vehicle | Ratio | bGH Dose |
| H | 24.5 | NaBz(1.6)/Surf. B(0.12) | GTS(73.8) | Miglyol/ Soya Oil | 1/2.5 | 175 mg day 0, day 7 |
| I | 24.5 | NaBz(1.6)/Surf. B(0.12) | GTS(73.8) | Miglyol/ Soya Oil (4/1) | 1/2.5 | 350 mg day 0 |
| J | -- | -- | -- | CBS | -- | 25 mg for 28 days |
| K | 7.5 | -- | GTS/Beeswax (9/1)(92.5) | saline | 1/5 | 750 mg day 0 |

Table VI

| Average Milk Production Increases from Dairy Cow Experiments | | | | |
|---|---|---|---|---|
| Formulation Number | Percent Increase in Milk Production Compared to Undosed Control Cows | | | |
| | Week 1 | Week 2 | Week 3 | Week 4 |
| A | 10.5 | 13.9 | 12.3 | 14.7 |
| B | 16.4 | 21.1 | 19.2 | 20.8 |
| C | 17.8 | 19.4 | 17.3 | 19.3 |
| D | 9.2 | 16.7 | 15.6 | 15.8 |
| E | 8.4 | 7.3 | 16.0 | 4.0 |
| F | 6.7 | 4.5 | 13.8 | 9.8 |
| G | 9.6 | 19.3 | 9.5 | 3.7 |
| H | 7.4 | 14.0 | 6.0 | 4.0 |
| I | 11.1 | 10.6 | 4.1 | 6.3 |
| J | 14.2 | 13.1 | 20.1 | 24.5 |
| K | 17.7 | 4.9 | 3.6 | 0.1 |

Table VII

| Average Plasma bGH Concentration (ng/mL by Radioimmunoassay) for Dairy Cow Experiments | | | | | | |
|---|---|---|---|---|---|---|
| Elapsed Time (Days) | Formulation Number | | | | | |
| | A[*] | B | C | D[*] | E | F |
| -24 Hr | 1.3 | 1.3 | 1.5 | 1.2 | 1.0 | 1.1 |
| 0 Hr | 1.4 | 1.2[**] | 1.4[**] | 1.1 | 1.0[**] | 1.1[**] |
| 3 Hr | 6.1 | 2.0 | 3.3 | 20.0 | 25.6 | 1.1 |
| 6 Hr | 5.2 | 6.9 | 8.5 | 7.2 | 50.1 | 1.2 |
| 1 Day | 2.6 | 30.8 | 37.6 | 1.5 | 13.5 | 13.8 |
| 2 Day | 2.4 | 9.7 | 20.1 | 3.7 | 7.2 | 8.4 |
| 5 Day | 2.8 | 6.7 | 9.2 | 3.9 | 8.2 | 5.8 |
| 7 Day | 2.9 | 7.3 | 9.9 | 7.4 | 8.2 | 6.9 |
| 9 Day | 3.4 | 7.2 | 9.8 | 7.8 | 8.5 | 7.3 |
| 12 Day | 3.7 | 6.4 | 8.5 | 3.8 | 2.9 | 1.8 |
| 14 Day | 2.7 | 4.3[**] | 5.6[**] | 4.0 | 2.4[**] | 1.5[**] |
| 16 Day | 3.2 | 14.2 | 21.2 | 5.5 | 45.7 | 15.7 |
| 19 Day | 3.9 | 9.9 | 11.2 | 7.2 | 9.6 | 9.0 |
| 21 Day | 3.2 | 14.7 | 12.9 | 6.4 | 8.1 | 7.5 |
| 23 Day | 2.4 | 9.5 | 8.0 | 6.3 | 7.8 | 6.1 |
| 26 Day | 2.5 | 7.8 | 8.4 | 5.4 | 5.4 | 3.7 |
| 28 Day | 2.2 | 4.6 | 6.1 | 4.9 | 5.0 | 2.8 |

[*]note: blood samples taken prior to daily injection
[**] Injections administered on the morning of these days after the blood sample was taken.

Table VII (Continued)

| Average Plasma bGH Concentration (ng/mL by Radioimmunoassay) for Dairy Cow Experiments | | | | | | |
|---|---|---|---|---|---|---|
| Elapsed Time (Days) | Formulation Number | | Elapsed Time (Days) | Formulation Number | | |
| | J* | K | | G* | H | I |
| -24 Hr | 2.3 | 5.2 | -24 Hr | 3.0 | 5.3 | 3.1 |
| 0 Hr | 4.1 | 7.3** | 0 Hr | 2.9 | 3.8** | 3.5** |
| 1 Hr | 12.2 | 34.5 | 3 Hr | 15.0 | 4.6 | 3.8 |
| 2 Hr | 15.3 | 57.2 | 6 Hr | 10.4 | 3.7 | 4.7 |
| 4 Hr | 15.8 | 127.8 | 1 Day | 5.1 | 10.2 | 31.5 |
| 6 hr | 12.7 | 102.7 | 2 Days | 4.7 | 7.8 | 24.5 |
| 1 Day | 4.4 | 107.7 | 3 Days | 4.2 | 6.2 | 8.4 |
| 2 Days | 4.0 | 33.3 | 4 Days | 4.8 | 6.7 | 9.2 |
| 3 Days | 4.1 | 15.3 | 5 Days | 4.3 | 6.5 | 9.7 |
| 4 Days | 4.7 | 13.1 | 6 Days | 6.1 | 8.0 | 11.2 |
| 6 Days | 5.7 | 15.8 | 7 Days | 6.2 | 9.6** | 13.1 |
| 8 Days | 6.0 | 17.6 | 8 Days | 6.0 | 18.0 | 11.9 |
| 11 Days | 6.7 | 34.5 | 10 Days | 6.5 | 16.8 | 13.1 |
| | | | 13 Days | 7.9 | 18.6 | 11.8 |
| | | | 17 Days | 5.2 | 16.5 | 11.0 |
| | | | 20 Days | 5.7 | 17.3 | 12.6 |

*note: blood samples taken prior to daily injection
** Injections administered on the morning of these days after the blood sample was taken.

EXAMPLE 4

Sustained release of compositions of the invention in sheep

Utilizing essentially the same procedure as Example 3, the compositions prepared in Example 1 and listed in Table VIII below are administered to groups of sheep (three animals per group). The animals are dosed with 3 mg bGH equivalents of microspheres per kg body weight. Daily blood samples are obtained for a 30 day period and assayed for bGH blood levels as previously described. The results of these experiments which are summarized in Table VIII below; demonstrate the effectiveness of the compositions of the invention for maintaining elevated levels of growth hormone in the blood for extended periods of time.

EP 0 257 368 B1

Table VIII (Continued)

Plasma levels by RIA in Sheep

| Composition | # Vehicle | Plasma level data | | | |
|---|---|---|---|---|---|
| | | Highest Level ng/ml | (hr) | Duration in (days) >10ng/mL | >5ng/mL |
| 8 | Soya oil | 290 | 24 | 10 | >24 |
| 8 | Soya oil/Miglyol 812 (1:1) | 290 | 24 | 6 | 13 |
| 34 | Soya oil/Miglyol 812 (1:1) | -- | 4-24 | 10 | >20 |
| 45 | " | -- | 4-24 | 3 | 6 |
| 46 | " | -- | 4-24 | 3 | 4 |
| 47 | " | -- | 4-24 | 3 | 10 |
| 43 | " | -- | 4-24 | 3 | 10 |

Table VIII

Plasma levels by RIA in Sheep

| Composition # | Vehicle | Highest Level ng/ml | (hr) | Duration in (days) >10ng/mL | >5ng/mL |
|---|---|---|---|---|---|
| 13 | 5%PEG(18,500MW) in Saline | 200 | 4 | 1 | 2 |
| 14 | " | 300 | 4 | 2 | 5 |
| 15 | 5%PEG(5000)MW in Saline | 140 | 6 | 1 | 2 |
| 15 | GDO | 50 | 6-24 | 2 | 3 |
| 32 | Miglyol 812 | 30 | 24 | 13 | 20 |
| 32 | Soya oil | 10 | 24 | >20 | >27 |
| 34 | Soya oil | 50 | 24 | 10 | 22 |
| 32 | GDO | 50 | 24 | 10 | 13 |
| 34 | Miglyol 812 | 100 | 6-24 | 10 | 22 |

EXAMPLE 5

Efficacy of bovine growth hormone mixture of the invention in dairy cows

A mixture of bovine growth hormone containing sodium benzoate (7% w/w) and a block copolymer of ethylene oxide and propylene oxide (0.5% w/w) is prepared by the spray drying procedure described in example 1.1.

The thus prepared mixture (7.28g) is added to a stirred solution of glyceryl tristearate (19.5g) in a 4/1 w/w mixture of, a mixture of caprylic, capric, lauric and caproic triglycerides (Miglyol® 812)[3]/soya oil (71 mL), at 75°C to 80°C. The mixture is stirred at 75°C to 80°C until it is homogeneous and is then cooled.

20

The resulting composition which is comprised on a weight basis of: bovine growth hormone 6.96%, sodium benzoate 0.51%, surfactant (block copolymer) 0.03% and vehicle 73.0%, is administered to lactating cows by the procedure of example 3. The results of these experiments which are summarized in Table IX below demonstrate the effectiveness of these compositions for increasing milk production for extended periods of time

Table IX

| Average Milk Production Increases Compared to Undosed Control Cows | |
|---|---|
| % increase Week 1 | % increase Week 2 |
| 15.7 | 12.0 |

**Claims**

1. A composition comprising on a weight basis about 2% to 70% growth hormone, somatomedin, growth factor or biologically active fragment or derivative thereof uniformly dispersed in about 30% to 95% fat or wax or mixture thereof, wherein said composition is provided in the form of microspheres.

2. A composition according to Claim 1 comprising on a weight basis about 40% to 95% wax or fat or mixture thereof; about 5% to 40% growth hormone, somatomedin, growth factor or biologically active fragment or derivative thereof; 0% to about 20% surfactant, preservative, stabilizer, salt, buffer or mixture thereof; wherein the weight average particle size of the composition is in a range of 25 microns to 300 microns.

3. A composition according to Claim 2 wherein the growth hormone is bovine, porcine or avian growth hormone.

4. A composition according to Claim 3 comprising on a weight basis about 20% to 40% bovine growth hormone; and about 55% to 79% glyceryl tristearate.

5. A composition according to Claim 4 for parenteral administration and slow release in a pharmaceutically and pharmacologically acceptable liquid vehicle.

6. A composition comprising on a weight basis about 20% to 40% bovine growth hormone; about 55% to 79% glyceryl tristearate; 0 to about 0.2% microbiological preservative; 0 to about 1% non-ionic surfactant; and 0 to about 2% sodium benzoate.

7. A method for increasing milk production in lactating animals comprising parenterally
   administering to the animal a composition comprising on a weight basis about 2% to 70% growth hormone, somatomedin, growth factor or biologically active fragment or derivative thereof uniformly dispersed in about 30% to 95% fat or wax or mixture thereof, wherein said composition is provided in the form of microspheres dispersed in a pharmaceutically and pharmacologically acceptable liquid vehicle.

8. A method according to Claim 7 wherein the composition comprises on a weight basis about 40% to 95% wax or fat or mixture thereof; about 5% to 40% animal growth hormone, 0% to about 20% surfactant, preservative, stabilizer, salt, buffer or mixture thereof and the weight average particle size of the composition is in a range of 25 microns to 300 microns.

9. A method according to Claim 8 wherein the composition comprises on a weight basis about 20% to 40% bovine growth hormone; about 55% to 79% glyceryl tristearate and the vehicle is a neutral mono-, di-, or triglyceride liquid or mixtures thereof.

10. A method for increasing weight gains in animals comprising parenterally

EP 0 257 368 B1

administering to the animal a composition comprising on a weight basis about 2% to 70% growth hormone, somatomedin, growth factor or biologically active fragment or derivative thereof uniformly dispersed in about 30% to 95% fat or wax or mixture thereof, wherein said composition is provided in the form of microspheres dispersed in a pharmaceutically and pharmacologically acceptable liquid vehicle.

11. A method according to Claim 10 wherein the composition comprises on a weight basis about 40% to 95% fat or wax or mixture thereof; about 5% to 40% animal growth hormone; 0% to about 20% surfactant, preservative, stabilizer, salt, buffer or mixture thereof; and the weight average particle size of the composition is in a range of 25 microns to 300 microns.

12. A method according to Claim 11 wherein the composition comprises on a weight basis about 20% to 40% bovine growth hormone; about 55% to 79% glyceryl tristearate; and the vehicle is a neutral mono-, di-, or triglyceride liquid or mixtures thereof.

13. A method for elevating and maintaining elevated blood levels of a growth hormone, somatomedin, growth factor or biologically active fragment or derivative thereof, improving feed efficiency, increasing growth rate, increasing muscle size, decreasing body fat and improving lean meat to fat ratio in an animal comprising parenterally
administering to the animal a composition comprising on a weight basis about 2% to 70% growth hormone, somatomedin, growth factor or biologically active fragment or derivative thereof uniformly dispersed in about 30% to 95% fat or wax or mixture thereof, wherein said composition is provided in the form of microspheres dispersed in a pharmaceutically and pharmacologically acceptable liquid vehicle.

14. A method according to Claim 13 wherein the composition comprises on a weight basis about 40% to 95% wax or a fat or mixture thereof; about 5% to 40% animal growth hormone; 0% to about 20% surfactant preservative, stabilizer, salt, buffer or mixture thereof and the weight average particle size of the composition is in a range of 25 microns to 300 microns.

15. A method according to Claim 14 wherein the composition comprises on a weight basis about 20% to 40% bovine growth hormone; about 55% to 79% glyceryl tristearate; and the vehicle is a neutral mono-, di-, or triglyceride liquid or mixtures thereof.

16. A slow release composition comprising growth hormone, somatomedin, growth factor or biologically active fragment or derivative thereof dispersed in fat or wax or mixture thereof dispersed in a pharmaceutically and pharmacologically acceptable liquid vehicle.

17. A composition according to Claim 16 characterized by heating about 1% to 30% growth hormone, somatomedin, growth factor or biologically active fragment or derivative thereof; about 5% to 60% fat or wax or mixture thereof; 0% to about 15% surfactant, preservative, stabilizer, salt, buffer or mixture thereof; and sufficient oil to total 100% to obtain a homogeneous mixture; and cooling said mixture to ambient temperature to form said composition.

18. A composition according to Claim 17 wherein the growth hormone is bovine, porcine or avian growth hormone.

19. A composition according to claim 18 comprising on a weight basis about 5% to 27% of bovine growth hormone; about 10% to 48% of glyceryl tristearate; 0% to about 0.2% microbiological preservative and 0% to about 1% non-ionic surfactant; and sufficient neutral mono-, di-, or triglyceride liquid or mixtures thereof to total 100%.

20. A method for increasing milk production in lactating animals comprising parenterally
administering to the animal a biologically active slow release composition characterized by heating about 1% to 30% growth hormone, somatomedin, growth factor or biologically active fragment or derivative thereof; about 5% to 60% fat or wax or mixture thereof; 0% to about 15% surfactant, preservative, stabilizer, salt, buffer or mixture thereof; and sufficient oil to total 100% to abtain a homogeneous mixture; and cooling said mixture to ambient temperature to form said composition.

22

**21.** A method for increasing milk production in animals according to Claim 20 wherein the composition comprises on a weight basis about 5% to 27% bovine growth hormone; about 10% to 48% glyceryl tristearate; 0% to about 0.2% microbiological preservative and 0% to about 1% non-ionic surfactant; and sufficient neutral mono-, di-, or triglyceride liquid or mixtures thereof to total 100%.

**22.** A method for increasing weight gains comprising parenterally

administering to the animal a biologically active slow release composition characterized by heating about 1% to 30% growth hormone, somatomedin, growth factor or biologically active fragment or derivative thereof; about 5% to 60% fat or wax or mixture thereof; 0% to about 15% surfactant, preservative, stabilizer, salt, buffer or mixture thereof; and sufficient oil to total 100% to obtain a homogeneous mixture; and cooling said mixture to ambient temperature to form said composition.

**23.** A method according to claim 22 wherein the composition comprises on a weight basis about 5% to 27% bovine growth hormone; about 10% to 48% of glyceryl tristearate; 0% to about 0.2% microbiological preservative and 0% to about 1% non-ionic surfactant; and sufficient neutral mono-, di-, or triglyceride liquid or mixtures thereof to total 100%.

**24.** A method for elevating and maintaining elevated blood levels of growth hormone, somatomedin, growth factor or biologically active fragment or derivative thereof, improving feed efficiency, increasing growth rate, increasing muscle size, decreasing body fat and improving lean meat to fat ratio in an animal comprising parenterally

administering to the animal a biologically active slow release composition characterized by heating about 1% to 30% growth hormone, somatomedin, growth factor or biologically active fragment or derivative thereof; about 5% to 60% fat or wax or mixture thereof; 0% to about 15% surfactant, preservative, stabilizer, salt, buffer or mixture thereof; and sufficient oil to total 100% to obtain a homogeneous mixture; and cooling said mixture to ambient temperature to form said composition.

**25.** A method according to Claim 24 wherein the composition comprises on a weight basis about 5% to 27% bovine growth hormone; about 10% to 48% of glyceryl tristearate; 0% to about 0.2% microbiological preservative and 0% to about 1% non-ionic surfactant; and sufficient neutral mono-, di-, or triglyceride liquid or mixtures thereof to total 100%.

**Revendications**

**1.** Composition comprenant, sur base pondérale, environ 2 % à 70 % d'hormone de croissance, de somatomédine, de facteur de croissance ou d'un fragment ou dérivé biologiquement actif de ceux-ci, en dispersion homogène dans environ 30 % à 95 % d une graisse ou d'une cire, ou d'un mélange de celles-ci, ladite composition étant fournie sous la forme de microsphères.

**2.** Composition selon la revendication 1, comprenant, sur base pondérale, environ 40 % à 95 % de cire ou de graisse, ou de leur mélange ; environ 5 % à 40 % d'hormone de croissance, de somatomédine, de facteur de croissance ou de leur fragment ou dérivé biologiquement actif ; 0 % à environ 20 % d un agent tensio-actif, conservateur, stabilisant, sel, tampon ou mélange d'entre eux ; la dimension granulométrique moyenne en poids de la composition se situant dans l'intervalle de 25 micromètres à 300 micromètres.

**3.** Composition selon la revendication 2, dans laquelle l'hormone de croissance est une hormone de croissance bovine, porcine ou aviaire.

**4.** Composition selon la revendication 3, comprenant, sur base pondérale, environ 20 % à 40 % d'hormone de croissance bovine ; et environ 55 % à 79 % de tristéarate de glycéryle.

**5.** Composition selon la revendication 4, destinée à une administration parentérale et une libération lente, dans un véhicule liquide pharmaceutiquement et pharmacologiquement acceptable.

**6.** Composition comprenant, sur base pondérale, environ 20 % à 40 % d'hormone de croissance bovine ; environ 55 % à 79 % de tristéarate de glycéryle ; 0 à environ 0,2 % de conservateur antimicrobien ; 0 à environ 1 % d'agent tensioactif non ionique ; et 0 à environ 2 % de benzoate de sodium.

7. Procédé pour augmenter la production de lait chez des animaux en lactation, comprenant l'administration parentérale à l'animal d'une composition comprenant, sur une base pondérale, environ 2 % à 70 % d'hormone de croissance, de somatomédine, de facteur de croissance ou d'un fragment ou dérivé biologiquement actif de ceux-ci, en dispersion uniforme dans environ 30 % à 95 % de graisse ou de cire, ou d'un mélange de celles-ci, ladite composition étant fournie sous la forme de microsphères dispersées dans un véhicule liquide pharmaceutiquement et pharmacologiquement acceptable.

8. Procédé selon la revendication 7, dans lequel la composition contient, sur base pondérale, environ 40 % à 95 % de cire ou de graisse, ou de leur mélange ; environ 5 % à 40 % d'hormone de croissance animale, 0 % à environ 20 % d'un agent tensio-actif, conservateur, stabilisant, sel, tampon ou mélange d'entre eux, et la dimension granulométrique moyenne en poids de la composition se situe dans l'intervalle de 25 micromètres à 300 micromètres.

9. Procédé selon la revendication 8, dans lequel la composition comprend, sur base pondérale, environ 20 % à 40 % d'hormone de croissance bovine ; environ 55 % à 79 % de tristéarate de glycéryle, et le véhicule est un mono-, di- ou triglycéride liquide neutre ou un mélange de ceux-ci.

10. Procédé pour augmenter les gains de poids chez des animaux, comprenant l'administration parentérale à l'animal d'une composition comprenant, sur base pondérale, environ 2 % à 70 % d'hormone de croissance, de somatomédine, de facteur de croissance ou d'un fragment ou dérivé biologiquement actif de ceux-ci, en dispersion uniforme dans environ 30 % à 95 % de graisse ou de cire, ou d'un mélange de celles-ci, ladite composition étant fournie sous la forme de microsphères dispersées dans un véhicule liquide pharmaceutiquement et pharmacologiquement acceptable.

11. Procédé selon la revendication 10, dans lequel la composition comprend, sur base pondérale, environ 40 % à 95 % de graisse ou de cire, ou de leur mélange ; environ 5 % à 40 % d'hormone de croissance animale ; 0 % à environ 20 % d un agent tensio-actif, conservateur, stabilisant, sel, tampon ou mélange d'entre eux ; et la dimension granulométrique moyenne en poids de la composition se situe dans l'intervalle de 25 micromètres à 300 micromètres.

12. Procédé selon la revendication 11, dans lequel la composition comprend, sur base pondérale, environ 20 % à 40 % d'hormone de croissance bovine ; environ 55 % à 79 % de tristéarate de glycéryle ; et le véhicule est un mono-, diou triglycéride liquide neutre ou un mélange de ceux-ci.

13. Procédé pour élever et maintenir des taux sanguins élevés d'hormone de croissance, de somatomédine, de facteur de croissance ou d'un fragment ou dérivé biologiquement actif de ceux-ci, améliorer le rendement d'alimentation, augmenter le rythme de croissance, augmenter la taille des muscles, diminuer les graisses corporelles et améliorer le rapport de la viande maigre à la graisse chez un animal, comprenant l'administration parentérale à l'animal d une composition comprenant, sur base pondérale, environ 2 % à 70 % d'hormone de croissance, de somatomédine, de facteur de croissance ou d'un fragment ou dérivé biologiquement actif de ceux-ci, en dispersion uniforme dans environ 30 % à 95 % de graisse ou de cire, ou d'un mélange de celles-ci, ladite composition étant fournie sous la forme de microsphères dispersées dans un véhicule liquide pharmaceutiquement et pharmacologiquement acceptable.

14. Procédé selon la revendication 13, dans lequel la composition comprend, sur base pondérale, environ 40 % à 95 % de cire ou de graisse, ou de leur mélange ; environ 5 % à 40 % d'hormone de croissance animale ; 0 % à environ 20 % d'un agent tensio-actif, conservateur, stabilisant, sel, tampon ou mélange d'entre eux, et la dimension granulométrique moyenne en poids de la composition se situe dans l'intervalle de 25 micromètres à 300 micromètres.

15. Procédé selon la revendication 14, dans lequel la composition comprend, sur base pondérale, environ 20 % à 40 % d'hormone de croissance bovine ; environ 55 % à 79 % de tristéarate de glycéryle ; et le véhicule est un mono-, di- ou triglycéride liquide neutre ou un mélange de ceux-ci.

16. Composition à libération lente comprenant une hormone de croissance, une somatomédine, un facteur de croissance ou un fragment ou dérivé biologiquement actif de ceux-ci, en dispersion dans une graisse, une cire ou un mélange de celles-ci, en dispersion dans un véhicule liquide pharmaceutique-

EP 0 257 368 B1

ment et pharmacologiquement acceptable.

17. Composition selon la revendication 16, caractérisée en ce que l'on chauffe d'environ 1 % à 30 % d'hormone de croissance, de somatomédine, de facteur de croissance ou d'un fragment ou dérivé biologiquement actif de ceux-ci ; environ 5 % à 60 % de la graisse ou cire ou de leur mélange ; 0 % à environ 15 % d'un agent tensio-actif, conservateur, stabilisant, sel, tampon ou mélange d'entre eux ; et une quantité suffisante d'huile pour faire un total de 100 %, de façon à obtenir un mélange homogène, et on refroidit ledit mélange à la température ambiante pour former ladite composition.

18. Composition selon la revendication 17, dans laquelle l'hormone de croissance est une hormone de croissance bovine, porcine ou aviaire.

19. Composition selon la revendication 18, comprenant, sur base pondérale, environ 5 % à 27 % d'hormone de croissance bovine ; environ 10 % à 48 % de tristéarate de glycéryle ; 0 % à environ 0,2 % de conservateur antimicrobien et 0 % à environ 1 % d'agent tensio-actif non ionique ; et une quantité suffisante d'un mono-, di- ou triglycéride liquide neutre, ou d'un mélange de ceux-ci, pour faire un total de 100 %.

20. Procédé pour augmenter la production de lait chez des animaux en lactation, comprenant l'administration parentérale à l'animal d une composition biologiquement active à libération lente, caractérisé en ce que l'on chauffe environ 1 % à 30 % d'hormone de croissance, de somatomédine, de facteur de croissance ou d'un fragment ou dérivé biologiquement actif de ceux-ci ; environ 5 % à 60 % de graisse ou de cire, ou d'un mélange de celles-ci ; 0 % à environ 15 % d'un agent tensio-actif, conservateur, stabilisant, sel, tampon ou mélange d'entre eux ; et une quantité suffisante d'huile pour faire un total de 100 %, de façon à obtenir un mélange homogène, et on refroidit ledit mélange à la température ambiante pour former ladite composition.

21. Procédé pour augmenter la production de lait chez des animaux selon la revendication 20, dans lequel la composition comprend, sur base pondérale, environ 5 % à 27 % d'hormone de croissance bovine ; environ 10 % à 48 % de tristéarate de glycéryle ; 0 % à environ 0,2 % de conservateur antimicrobien et 0 % à environ 1 % d'agent tensioactif non ionique ; et une quantité suffisante d'un mono-, di- ou triglycéride liquide neutre, ou d'un mélange de ceuxci, pour faire un total de 100 %.

22. Procédé pour augmenter les gains de poids chez des animaux, comprenant l'administration parentérale à l'animal d'une composition biologiquement active à libération lente, caractérisé en ce que l'on chauffe environ 1 % à 30 % d'hormone de croissance, de somatomédine, de facteur de croissance ou d'un fragment ou dérivé biologiquement actif de ceux-ci ; environ 5 % à 60 % de graisse ou de cire, ou d'un mélange de celles-ci, 0 % à environ 15 % d'un agent tensio-actif, conservateur, stabilisant, sel, tampon ou mélange d'entre eux ; et une quantité suffisante d'huile pour faire un total de 100 %, de façon à obtenir un mélange homogène, et on refroidit ledit mélange à la température ambiante pour former ladite composition.

23. Procédé selon la revendication 22, dans lequel la composition comprend, sur base pondérale, environ 5 % à 27 % d'hormone de croissance bovine ; environ 10 % à 48 % de tristéarate de glycéryle ; 0 % à environ 0,2 % de conservateur antimicrobien ; et 0 % à environ 1 % d'agent tensio-actif non ionique ; et une quantité suffisante d'un mono-, di- ou triglycéride liquide neutre, ou d'un mélange de ceux-ci, pour faire un total de 100 %.

24. Procédé pour élever et maintenir des taux sanguins élevés d'hormone de croissance, de somatomédine, de facteur de croissance ou d'un fragment ou dérivé biologiquement actif de ceux-ci, améliorer le rendement d'alimentation, augmenter le rythme de croissance, augmenter la taille des muscles, diminuer les graisses corporelles et améliorer le rapport de la viande maigre à la graisse chez un animal, comprenant l'administration parentérale à l'animal d'une composition à biologiquement active libération lente, caractérisé en ce que l'on chauffe environ 1 % à 30 % d'hormone de croissance, de somatomédine, de facteur de croissance ou d'un fragment ou dérivé biologiquement actif de ceux-ci ; environ 5 % à 60 % de graisse ou de cire, ou d'un mélange de celles-ci ; 0 % à environ 15 % d'un agent tensio-actif, conservateur, stabilisant, sel, tampon ou mélange d'entre eux ; et une quantité suffisante d'huile pour faire un total de 100 %, de façon à obtenir un mélange homogène, et on refroidit

25

ledit mélange à la température ambiante pour former ladite composition.

**25.** Procédé selon la revendication 24, dans lequel la composition comprend, sur base pondérale, environ 5 % à 27 % d'hormone de croissance bovine ; environ 10 % à 48 % de tristéarate de glycéryle ; 0 % à environ 0,2 % de conservateur antimicrobien ; et 0 % à environ 1 % d'agent tensio-actif non ionique ; et une quantité suffisante d'un mono-, di- ou triglycéride liquide neutre ou d'un mélange de ceux-ci, pour faire un total de 100 %.

**Patentansprüche**

**1.** Zusammensetzung, umfasssend auf Gewichtsbasis etwa 2 bis 70 % Wachstumshormon, Somatomedin, Wachstumsfaktor oder biologisch aktives Fragment oder Derivat derselben, einförmig dispergiert in etwa 30 bis 95 % Fett oder Wachs oder Gemisch derselben, wobei die Zusammensetzung in Form von Mikrosphären vorliegt.

**2.** Zusammensetzung gemäß Anspruch 1, umfassend auf Gewichtsbasis etwa 40 bis 95 % Wachs oder Fett oder Gemisch derselben; etwa 5 bis 40 % Wachstumshormon, Somatomedin, Wachstumsfaktor oder biologisch aktives Fragment oder Derivat derselben; 0 bis etwa 20 % Surfaktans, Konservierungsstoff, Stabilisator, Salz, Puffer oder Gemisch derselben, wobei das Gewichtsmittel der Teilchengröße der Zusammensetzung in einem Bereich von 25 $\mu$m bis 300 $\mu$m liegt.

**3.** Zusammensetzung gemäß Anspruch 2, wobei es sich bei dem Wachstumshormon um Rinder-, Schweine- oder Geflügelwachstumshormon handelt.

**4.** Zusammensetzung gemäß Anspruch 3, umfassend auf Gewichtsbasis etwa 20 bis 40 % Rinderwachstumshormon; sowie etwa 55 bis 79 % Glyceryltristearat.

**5.** Zusammensetzung gemäß Anspruch 4, zur parenteralen Verabreichung und langsamen Freigabe in ein pharmazeutisch und pharmakologisch akzeptables flüssiges Vehikel.

**6.** Zusammensetzung, umfassend auf Gewichtsbasis etwa 20 bis 40 % Rinderwachstumshormon; etwa 55 bis 79 % Glyceryltristearat; 0 bis etwa 0,2 % mikrobiologisches Konservierungsmittel; 0 bis etwa 1 % nicht-ionisches Surfaktans und 0 bis etwa 2 % Natriumbenzoat.

**7.** Verfahren zur Steigerung der Milcherzeugung in laktierenden Tieren, wobei man dem Tier parenteral eine Zusammensetzung verabreicht, umfassend auf Gewichtsbasis etwa 2 bis 70 % Wachstumshormon, Somatomedin, Wachstumsfaktor oder biologisch aktives Fragment oder Derivat derselben, einförmig dispergiert in etwa 30 bis 95 % Fett oder Wachs oder Gemisch derselben, wobei die Zusammensetzung in Form von Mikrosphären bereitgestellt wird, die in einem pharmazeutisch und pharmakologisch akzeptablen flüssigen Vehikel dispergiert sind.

**8.** Verfahren gemäß Anspruch 7, wobei die Zusammensetzung auf Gewichtsbasis etwa 40 bis 95 % Wachs oder Fett oder Gemisch derselben; etwa 5 bis 40 % tierisches Wachstumshormon, 0 bis etwa 20 % Surfaktans, Konservierungsmittel, Stabilisator, Salz, Puffer oder Gemisch derselben umfaßt und das Gewichtsmmittel der Teilchengröße der Zusammensetzung in einem Bereich von 25 $\mu$m bis 300 $\mu$m liegt.

**9.** Verfahren gemäß Anspruch 8, wobei die Zusammensetzung auf Gewichtsbasis etwa 20 bis 40 % Rinderwachstumshormon; etwa 55 bis 79 % Glyceryltristearat umfaßt und das Vehikel eine neutrale Mono-, Di- oder Triglyceridflüssigkeit oder Mischungen derselben ist.

**10.** Verfahren zur Steigerung der Gewichtszunahme bei Tieren, wobei man dem Tier eine Zusammensetzung parenteral verabreicht, welche auf Gewichtsbasis etwa 2 bis 70 % Wachstumshormon, Somatomedin, Wachstumsfaktor oder biologisch aktives Fragment oder Derivat derselben einförmig dispergiert in etwa 30 bis 95 % Fett oder Wachs oder Gemisch derselben umfaßt, wobei die Zusammensetzung in der Form von Mikrosphären bereitgestellt wird, die in einem pharmazeutisch und pharmakologisch akzeptablen flüssigen Vehikel dispergiert sind.

**11.** Verfahren gemäß Anspruch 10, wobei die Zusammensetzung auf Gewichtsbasis etwa 40 bis 95 % Fett oder Wachs oder Gemisch derselben; etwa 5 bis 40 % tierisches Wachstumshormon; 0 bis etwa 20 % Surfaktans, Konservierungsmittel, Stabilisator, Salz, Puffer oder Gemisch derselben umfaßt; und das Gewichtsmittel der Teilchengröße der Zusammensetzung in einem Bereich von 25 $\mu$m bis 300 $\mu$m liegt.

**12.** Verfahren gemäß Anspruch 11, wobei die Zusammensetzung auf Gewichtsbasis etwa 20 bis 40 % Rinderwachstumshormon; etwa 55 bis 79 % Glyceryltristearat umfaßt; und es sich bei dem Vehikel um eine neutrale Mono-, Di-, oder Triglyceridflüssigkeit oder Mischungen derselben handelt.

**13.** Verfahren zur Erhöhung und Aufrechterhaltung erhöhter Blutspiegel von einem Wachstumshormon, Somatomedin, Wachstumsfaktor oder biologisch aktivem Fragment oder Derivat derselben, zur Verbesserung der Futtereffizienz, Steigerung der Wachstumsrate, Steigerung der Muskelgröße, Verringerung des Körperfetts und Verbesserung des Fleisch-zu-Fett-Verhältnisses bei einem Tier, wobei dem Tier eine Zusammensetzung parenteral verabreicht wird, welche auf Gewichtsbasis etwa 2 bis 70 % Wachstumshormon, Somatomedin, Wachstumsfaktor und biologisch aktives Fragment oder Derivat derselben einförmig dispergiert in etwa 30 bis 95 % Fett oder Wachs oder Gemisch derselben umfaßt, wobei die Zusammensetzung in der Form von Mikrosphären bereitgestellt wird, die in einem pharmazeutisch und pharmakologisch akzeptablen flüssigen Vehikel dispergiert sind.

**14.** Verfahren gemäß Anspruch 13, wobei die Zusammensetzung auf Gewichtsbasis etwa 40 bis 95 % Wachs oder ein Fett oder Gemisch derselben umfaßt; etwa 5 bis 40 % tierisches Wachstumshormon; 0 bis etwa 20 % Surfaktans, Konservierungsmittel, Stabilisator, Salz, Puffer oder Gemisch derselben und das Gewichtsmittel der Teilchengröße der Zusammensetzung in einem Bereich von 25 $\mu$m bis 300 $\mu$m liegt.

**15.** Verfahren gemäß Anspruch 14, wobei die Zusammensetzung auf Gewichtsbasis etwa 20 bis 40 % Rinderwachstumshormon; etwa 55 bis 79 % Glyceryltristearat umfaßt und es sich bei dem Vehikel um eine neutrale Mono-, Di-, oder Triglyceridflüssigkeit oder Mischungen derselben handelt.

**16.** Zusammensetzung mit verzögerter Freigabe, umfassend Wachstumshormon, Somatomedin, Wachstumsfaktor oder biologisch aktives Fragment oder Derivat derselben, dispergiert in Fett oder Wachs oder Gemisch derselben, dispergiert in einem pharamzeutisch und pharmakologisch akzeptablen flüssigen Vehikel.

**17.** Zusammensetzung gemäß Anspruch 16, dadurch gekennzeichnet, daß man etwa 1 bis 30 % Wachstumshormon, Somatomedin, Wachstumsfaktor oder biologisch aktives Fragment oder Derivat derselben; etwa 5 bis 60 % Fett oder Wachs oder Gemisch derselben; 0 bis etwa 15 % Surfaktans, Konservierungsmittel, Stabilisator, Salz, Puffer oder Gemisch derselben; und ausreichend Öl, um die Gesamtmenge auf 100 % zu bringen, erhitzt, um eine homogene Mischung zu erhalten, und Abkühlen der Mischung auf Umgebungstemperatur zur Bildung der Zusammensetzung.

**18.** Zusammensetzung gemäß Anspruch 17, wobei es sich bei dem Wachstumshormon um Rinder-, Schweine- oder Geflügelwachstumshormon handelt.

**19.** Zusammensetzung gemäß Anspruch 18, umfassend auf Gewichtsbasis etwa 5 bis 27 % Rinderwachstumshormon; etwa 10 bis 48 % Glyceryltristearat; 0 bis 0,2 % mikrobiologisches Konservierungsmittel und 0 bis 1 % nicht-ionisches Surfaktans; sowie eine ausreichende Menge neutrale Mono-, Di- oder Triglyceridflüssigkeit oder Gemische derselben, um das Ganze auf 100 % zu bringen.

**20.** Verfahren zur Steigerung der Milcherzeugung bei laktierenden Tieren, wobei man dem Tier parenteral eine biologisch aktive Zusammensetzung mit verzögerter Freigabe verabreicht, die gekennzeichnet ist durch das Erhitzen von etwa 1 bis 30 % Wachstumshormon, Somatomedin, Wachstumsfaktor oder biologisch aktives Fragment oder Derivat derselben; etwa 5 bis 60 % Fett oder Wachs oder Gemisch derselben; 0 bis etwa 15 % Surfaktans, Konservierungsmittel, Stabilisator, Salz, Puffer, oder Gemisch derselben sowie ausreichend Öl, um die Gesamtmenge auf 100 % zu bringen, um ein homogenes Gemisch zu erhalten; und Abkühlung des Gemisches auf Umgebungstemperatur, um die Zusammensetzung zu bilden.

21. Verfahren zur Steigerung der Milcherzeugung bei Tieren gemäß Anspruch 20, wobei die Zusammensetzung auf Gewichtsbasis etwa 5 bis 27 % Rinderwachstumshormon; etwa 10 bis 48 % Glyceryltristearat; 0 bis etwa 0,2 % mikrobiologische Konservierungsstoffe und 0 bis etwa 1 % nicht-ionisches Surfaktans umfaßt, sowie ausreichend neutrale Mono-, Di- oder Triglyceridflüssigkeit oder Mischungen derselben, um die Gesamtmenge auf 100 % zu bringen.

22. Verfahren zur Steigerung von Gewichtszunahme, wobei man dem Tier eine biologisch aktive Zusammensetzung mit verzögerter Freigabe parenteral verabreicht, die gekennzeichnet ist durch Erhitzen von etwa 1 bis 30 % Wachstumshormon, Somatomedin, Wachstumsfaktor, biologisch aktivem Fragment oder Derivat derselben; etwa 5 bis 60 % Fett oder Wachs oder Gemisch derselben; 0 bis etwa 15 % Surfaktans, Konservierungsmittel, Stabilisator, Salz, Puffer oder Gemisch derselben; sowie ausreichend Öl, um die Gesamtmenge auf 100 % zu bringen, um eine homogene Mischung zu erhalten und Abkühlen der Mischung auf Umgebungstemperatur, um die Zusammensetzung zu bilden.

23. Verfahren gemäß Anspruch 22, wobei die Zusammensetzung auf Gewichtsbasis etwa 5 bis 27 % Rinderwachstumshormon; etwa 10 bis 48 % Glyceryltristearat; 0 bis etwa 0,2 % mikrobiologisches Konservierungsmittel und 0 bis 1 % nicht-ionisches Surfaktans umfaßt, sowie ausreichend neutrale Mono-, Di- oder Triglyceridflüssigkeit oder Gemische derselben, um die Gesamtmenge auf 100 % zu bringen.

24. Verfahren zur Erhöhung und Aufrechterhaltung erhöhter Blutspiegel an Wachstumshormon, Somatomedin, Wachstumsfaktor oder biologisch aktivem Fragment oder Derivat derselben, zur Verbesserung der Futtereffizienz, Steigerung der Wachstumsrate, Steigerung der Muskelgröße, Verringerung des Körperfetts und Verbesserung des Fleisch-zu-Fett-Verhältnisses in einem Tier, wobei man dem Tier eine biologisch aktive Zusammensetzung mit verzögerter Freigabe parenteral verabreicht, gekennzeichnet durch das Erhitzen von etwa 1 bis 30 % Wachstumshormon, Somatomedin, Wachstumsfaktor oder biologisch aktivem Fragment oder Derivat derselben; etwa 5 bis 60 % Fett oder Wachs oder Gemisch derselben; 0 bis etwa 15 % Surfaktans, Konservierungsmittel, Stabilisator, Salz, Puffer oder Gemisch derselben; sowie ausreichend Öl, um die Gesamtmenge auf 100 % zu bringen, um ein homogenes Gemisch zu erhalten, und Abkühlen der Mischung auf Umgebungstemperatur, um die Zusammensetzung zu bilden.

25. Verfahren gemäß Anspruch 24, wobei die Zusammensetzung auf Gewichtsbasis etwa 5 bis 27 % Rinderwachstumshormon; etwa 10 bis 48 % Glyceryltristearat; 0 bis etwa 0,2 % mikrobiologisches Konservierungsmittel und 0 bis etwa 1 % nicht-ionisches Surfaktans umfaßt; sowie ausreichend neutrale Mono-, Di- oder Triglyceridflüssigkeit oder Gemische derselben, um die Gesamtmenge auf 100 % zu bringen.